# EUROPEAN PATENT SPECIFICATION

(11) **EP 0 664 776 B1**
(45) Date of publication and mention of the grant of the patent: **10.07.1996**
(21) Application number: 93924214.5
(22) Date of filing: 12.10.1993
(51) Int. Cl.: C07C 6/04, B01J 23/92

(54) **PROCESS FOR THE CONVERSION OF AN OLEFIN OR A MIXTURE OF OLEFINS**
VERFAHREN ZUR UMSETZUNG VON OLEFINEN ODER VON EINER OLEFINENMISCHUNG
PROCEDE POUR LA CONVERSION D'UNE OLEFINE OU D'UN MELANGE D'OLEFINES

(30) Priority: 19.10.1992 NL 9201810
(43) Date of publication of application: 02.08.1995
(73) Proprietor: DSM N.V., NL-6411 TE Heerlen (NL)
(72) Inventor: VAN GEEM, Paul, Christiaan, NL-6174 RC Schinnen (NL); VAN HARDEVELD, Rudolf, NL-6191 BA Beek (NL); OONK, Johannes, NL-7333 BD Apeldoorn (NL); GOOSSENS, Henricus, Christiaan, Andrianus, NL-6417 HK Heerlen (NL)
(86) International application number: NL9300201
(87) International publication number: WO9408922

(56) References cited:
- EP-A- 0 139 774
- EP-A- 0 304 515
- FR-A- 2 254 369
- US-A- 3 660 506

## Description

The invention relates to a process for the conversion of an olefin or a mixture of olefins in a distillation column in which both a metathesis and an isomerization reaction occur.

Such a process is described in EP-A-304,515. In said patent publication olefins are converted into other olefins by means of a combination of a metathesis reaction and a double bond isomerization.

Here and hereinafter a metathesis reaction is understood to be a (catalytic) reaction in which double bonds in olefins are interchanged as a result of which equimolar amounts of new olefins are formed. In such a reaction, which moves towards equilibrium, a good approximation of statistical distribution of the alkylidene fragments of the olefin(s) involved over all possible combinations is obtained.

Isomerization is here and hereinafter understood to be the (catalytic) displacement of the double bond in an olefin. Also this type of reaction generally moves towards equilibrium.

By combining the said reactions it is possible to convert olefins into ethylene and propylene (which are highly desirable monomers for polymer chemical activities) and higher, linear alkenes.

In such a combined process use can be made of two separate catalysts which provide either metathesis or isomerization; this is described, inter alia, in NL-C-184.270. Use can also be made of a catalyst which combines both activities and of which examples are given in US-A-4,180,524.

The disadvantage of such processes is that ultimately said reactions reach equilibrium and do not lead to the maximum possible formation of the desired products ethylene and propylene, which therefore are obtained in only moderate yield.

The solution, as given in EP-A-304,515, is to perform the process in a reactive distillation column. By applying a physical separation of the reaction products during the process in the reactor, the desired products ethylene and propylene are withdrawn from the equilibrium. As a result, an increased yield of ethylene and propylene is achieved.

Applicant has found that even this increased yield of ethylene and propylene is relatively low and is still economically unattractive; the degree of conversion of the feedstock is also low. To increase the yield of ethylene and propylene and to improve the conversion, it has been found that this can be achieved by using a distillation column, where in the top section of the column a metathesis reaction takes place, while in the bottom section a combined metathesis and isomerization reaction takes place.

In such a situation it is preferred to feed the olefin or olefin mixture to be treated to the column at a point above the said bottom section. If the feed is directed otherwise, it will be in primary contact with the isomerisation catalyst, which will result in a loss of C1-fragments, needed for the production of ethylene and propylene.

An alternative is to carry out the process in a distillative metathesis reactor combined with a downstream distillative metathesis/isomerization reactor; as a result of which it is possible to choose different process conditions in the reactors (like lower pressure (and thus lower temperature) in the downstream reactor).

For other types of processes the use of a reactive distillation column as such is known. For more information on this point, EP-A-461,855 can be referred to, for example. On the basis of the physical properties of the olefin or mixture of olefins to be treated and the main products, one skilled in the art can determine the parameters (such as diameter, height of top and bottom section, reflux ratio, number of transfer units).

The catalysts to be applied in the process according to the invention are known per se. The number of catalysts suitable for a metathesis reaction, for example, is very large. Most of the catalytic systems to be used for this purpose are derived from transition metals from groups 4-10 of the Periodic System of the Elements (according to the latest IUPAC nomenclature); they can be applied in homogeneous as well as heterogeneous form.

Examples of homogeneous metathesis catalysts are ReCl₅ or W(CO)₆, whether or not in combination with a cocatalyst (such as, for example EtAlCl₂ or Ph₂C=W(CO)₅).

In a specific form of the present invention the top section of the distillation column is filled with inert packing (like Raschig Rings, Berl saddles or the like) and the metathesis catalyst is supplied in the form of a solution in an inert solvent (for instance an aromatic hydrocarbon, like (halo-)benzene or toluene). Another possibility is the supply of the catalyst in the form of a slurry in an adequate dispersant. The solution or dispersion, containing the metathesis catalyst, is fed to the top of the distillation column and trickles down the column. It can be removed from the column either from the bottom of the top section or even from the bottom of the bottom section, depending of the type of catalyst present in the bottom section (where the combined metathesis and isomerization reaction occurs). The choice of the solvent or dispersant has to be such that it is not so volatile to be distilled over the top of the column, but it is to be removed from the bottom. The metathesis catalyst can also be deposited as active material on a structurized material (structured packing).

As the active phase of heterogeneous metathesis catalysts, to be applied as a slurry or in the form of a packed bed, use is made predominantly of a supported metal oxide (such as MoO₃, Re₂O₇ or WO₃), whether or not in combination with activity-increasing components such as tetra-alkyl-tin and tetra-alkyl-lead. The support material, too, is important: the higher the Brönsted acid strength of the support, the higher the rate of reaction. In a large number of cases this acid nature of the catalyst also gives rise to double bond isomerization, which in metathesis literature has led to publications that indicate how such a side reaction can be prevented (J.C. Mol in J. Mol. Cat. 46, 1988, pp. 151-156).

The double bond isomerization is usually carried out in the presence of solid acids or solid bases. Examples of solid acids are silica-alumina, γ-alumina and the like; examples of solid bases are sodium on alumina, cesium containing zeolite and the like.

Since it is required that, according to the invention, besides a metathesis reaction, also isomerization occurs in the bottom part of the distillation column, in this section use is preferably made of a bifunctional catalyst, i.e. a catalyst that provides both a metathesis reaction and an isomerization reaction. As special example of such catalysts mention may be made of Re₂O₇ or MoO₃ on a silica-alumina support. Of course, also the use of a physical blend of a metathesis and an isomerization catalyst is viable.

The process according to the invention is particularly suitable for use in the conversion of butenes, pentenes and/or hexenes, and more preferably for a butene-containing stream. Particularly during the preparation of MTBE (methyl tertiary butyl ether) from C₄-hydrocarbon containing streams, originating from refinery processes or steam cracking, a large amount of so-called C₄-raffinate-2 is obtained, which contains a large amount of butenes; rather than selling or cracking such a stream, it can now be processed using a process according to the invention and give a high yield of ethylene and propylene. The resulting higher hydrocarbons, discharged from the bottom of the reactive distillation column, can be reused, for example as feed for a cracking process, so that a higher overall feedstock efficiency can be achieved.

The conditions under which the process of the invention is to be carried out are, in a general sense, known from metathesis and isomerization chemistry.

The temperature range, for example, will be between 0 and 250°C (preferably between 10 and 180°C) and the pressure will generally be higher than atmospheric pressure, and preferably between 0.2 and 5.0 MPa.

A preferable condition for successful operation of the process is the absence in the reaction system and in the feed of water, oxygen or polar components (such as amines or alcohols); their presence causes a dramatic decrease in the activity of the catalyst.

Applicant has found that when the aim is an increased propylene yield, the above mentioned conversion of an olefin or mixture of olefins can be improved by adding an extra olefin, in particular ethylene.

When using heterogeneous metathesis and isomerization catalysts in the form of a fixed bed, the particle size of the catalysts should preferably be between 200 µm and 10 mm. The amount of catalyst to be used in the top section of the distillation column (the metathesis-section) as well as the amount of catalyst(s) to be used in the bottom section of the distillation column (the metathesis + isomerization section) is related to the catalytic activity of said catalysts and the conversion/selectivity aimed at. The skilled man can easily determine the optimum conditions for this process. Applicant has found that through undesirable side reactions and by-reactions sometimes a decrease of the metathesis activity occurs and this deactivation cannot be eliminated using a reactivation method for such catalysts as known from literature. The method described by Amigues (Hydrocarbon Processing, Oct. 1990; pp. 79-80): air treatment (at 550°C) followed by activation in nitrogen (at 550°C), for example, has proved unworkable: the catalyst thus treated did not show much activity. Attempts to treat such a deactivated catalyst with air at higher temperatures (up to 800°C), followed by a nitrogen treatment at 550°C, also proved futile: the catalyst still did not exhibit much activity after such a treatment. Without claiming any scientific status for this explanation, this problem may be attributable to the fact that the combined metathesis and isomerization in such a reactive distillation leads to other by-products than in the conventional processes.

The invention therefore also aims to provide a process for the conversion of an olefin or a mixture of olefins in which said problem has also been solved.

Applicant has found that the catalysts, or catalyst combination used in combined metathesis and isomerization in a reactive distillation column as per the invention, can suitably be reactivated by periodically subjecting the catalyst, in succession, to treatment with a C₂-C₄ olefin (and preferably with ethylene or propylene), treatment with an oxygen-containing gas and treatment with a nitrogen-containing gas.

Surprisingly, it has been found that when a metathesis and isomerization process is carried out in a reactive distillation column, as per the invention, the catalyst being periodically reactivated in the specific manner of the invention, the result is an economically attractive process with a higher ethylene and propylene yield relative to the amount of the olefin feed.

In addition, the catalyst can suitably be reactivated, so that there is no need for costly exchange of the catalyst from the reactor.

Such a pre-treatment with a C₂-C₄ olefin, at pressures from 0.1-1.0 MPa and temperatures from 50-200°C, results in a catalyst which can subsequently be reactivated effectively by the Amigues method.

Reactivation of a deactivated homogeneous metathesis catalyst or of the slurry metathesis catalyst can also be performed outside the distillation column, in a separate treatment. This obviates the periodic reactivation of the contents of the distillation column.

It can be of advantage to submit the bottom stream of the distillation column to a co-metathesis with a lower α-olefin, preferably with ethylene or propylene and most preferred with ethylene. As a result of such an olefinolysis, especially such an ethenolysis, the higher boiling olefins of the bottom stream of the reactor (in majority being internal olefins) can be converted to lower α-olefins.
The process conditions for such an olefinolysis are in general the same as for the methathesis reaction. The lower α-olefin needed can be taken from the (workup of the) topstream of the distillation column. To avoid significant selfmetathesis of the olefins to be treated, a surplus of olefin (especially ethylene) relative to the higher boiling olefins from the bottom stream is applied. Generally a molar ratio of lower-α-olefin to higher-α-olefin of at least 2 is to be applied.

The invention will be illustrated by the following example and comparative experiment, without being limitative hereto.

### Example I

A reactive distillation column with a internal diameter of 8 x 10⁻² m and a height of 2.5 m was used having 25 distillation trays equally divided over the column. Tray 8 was used as feed tray. The top section of the column (trays 1-11) contained a metathesis catalyst, 5.0 wt.% molybdenum oxide on silica; the bottom section of the column (trays 13-25) contained a 1:1 mixture of the above metathesis catalyst with an isomerisation catalyst, 3.5 wt.% rutheniumoxide on alumina; both catalysts had an average diameter of about 1.5 mm; tray 12 was intentionally kept empty for separation purposes. Each catalyst tray contained about 100 grams of catalyst. The column was fed with 8.5 kg/hour of a mixture of 60 mol % of butene-1 and 40 mol % of butene-2. The top pressure was set at 3.0 MPa; the top temperature in the steady state was 397 K; the bottom temperature was 430 K. After analysis it was found that the conversion of the butenes was 37.3%; the ethylene yield was 197.6 grams/hour, the propylene yield was 902.1 grams/hour which corresponds to a selectivity of, resp., 12.5 and 38.0 mol %. The bottom stream contained mainly C₅ and C₆ olefins, in a selectivity of resp. 36.4 and 12.9 mol %.

### Comparative experiment A

The same setup was used as in Example 1, with the exception that all catalytic trays (1-11 and 13-25) contained a 1:1 mixture of the methathesis and the isomerisation catalyst, used in example I. The top temperature was 405 K; the bottom temperature was 424K. After analysis it was found that the conversion of the butenes was only 24.7 %. The ethylene yield was 63.7 grams/hour, the propylene yields was 701.2 grams/hour, which corresponds to a selectivity of, resp. 6.1 and 44.5 mol %. The bottomstream contained mainly C₅ and C₆ olefins, in a selectivity of resp. 42.0 and 7.2 mol %.

### Example II

Example I was repeated, with the exception that the feed was introduced on tray 12. The bottom temperature was 429 K, the top temperature 398 K. The butenes-conversion proved to be 35%; the ethylene yield was 148.7 grams/hour, the propylene yield 911,6 grams/hour. This corresponds to a selectivity of, resp., 9.9 and 40.8 mol %. The selectivity to C₅ and C₆-olefins in the bottomstream was, resp., 37.9 and 11.1 mol %.

### Comparative experiment B

Comparative experiment A was repeated, with the exception that the feed was introduced in tray 12. The top temperature was 404 K; the bottom temperature 425 K. The conversion of butenes was 25 mol %; the yield of ethylene and propylene was, resp., 61.6 and 714,0 grams/hour. This corresponds to a selectivity of 5.9 resp. 44.9 mol %. The bottomstream contained C₅ and C₆-olefins in a resp. selectivity of 41.8 and 7.2 mol %.

The above shows that a substantial increase of the conversion and thus the yield of ethylene and propylene can be obtained by using the process of the present invention.

## Claims

1. Process for the conversion of an olefin or a mixture of olefins in a distillation column in which both a metathesis and an isomerization reaction occur, characterized in that in the top section of the column a metathesis reaction takes place and that in the bottom section of the column a combined metathesis and isomerization reaction takes place.

2. Process according to claim 1, characterized in that the bottom section of the distillation column is filled with a bifunctional catalyst which provides both the isomerization reaction and the metathesis reaction.

3. Process according to claim 2, characterized in that rhenium oxide or molybdenum oxide on a silica-alumina support is applied as bifunctional catalyst.

4. Process according to claim 1, characterized in that a slurry or solution of a metathesis catalyst is fed to the top section of the column, wherein said top section is filled with inert packing.

5. Process according to anyone of claims 1-4, characterized in that the olefin or the mixture of olefins is fed to the distillation column at a point above the said bottom section.

6. Process according to anyone of claims 1-5, characterized in that the mixture of olefins comprises a mixture of butenes, pentenes or hexenes.

7. Process according to anyone of claims 1-6, characterized in that the mixture of olefins also contains ethylene.

8. Process according to any one of claims 1-7, characterized in that the catalyst is periodically subjected to reactivation, which comprises treatment, in succession, with a C₂-C₄ olefin, treatment with an oxygen-containing gas and treatment with a nitrogen-containing gas.

9. Process according to claim 8, characterized in that the reactivation is carried out using ethylene.

10. Process according to anyone of claims 8-9, characterized in that reactivation with the olefin is carried out at a temperature of 50-200°C.

11. Process according to anyone of claims 1-10, characterized in that the product of the bottom section of the distillation is submitted to a co-metathesis with a lower α-olefin.

12. Process according to claim 11, characterized in that as lower α-olefin ethylene is used.

## Patentansprüche

1. Verfahren zur Umwandlung eines Olefins oder einer Mischung von Olefinen in einer Destillationssäule, in der sowohl eine Metathese- als auch eine Isomerisierungsreaktion auftreten, dadurch gekennzeichnet, daß im oberen Abschnitt der Säule eine Metathesereaktion stattfindet, und daß im unteren Abschnitt der Säule eine kombinierte Metathese- und Isomerisierungsreaktion stattfindet.

2. Verfahren nach Anspruch 1, dadurch gekennzeichnet, daß der untere Abschnitt der Destillationssäule mit einem bifunktionellen Katalysator gefüllt wird, der sowohl die Isomerisierungsreaktion als auch die Metathesereaktion vorsieht.

3. Verfahren nach Anspruch 2, dadurch gekennzeichnet, daß Rheniumoxid oder Molybdänoxid auf einem Kieselerde-Tonerde-Träger als bifunktioneller Katalysator verwendet wird.

4. Verfahren nach Anspruch 1, dadurch gekennzeichnet, daß eine Aufschlämmung oder Lösung eines Metathesekatalysators dem oberen Abschnitt der Säule zugeführt wird, wobei der obere Abschnitt mit einer inerten Packung gefüllt ist.

5. Verfahren nach einem der Ansprüche 1 bis 4, dadurch gekennzeichnet, daß das Olefin oder die Mischung von Olefinen der Destillationssäule an einem Punkt oberhalb des unteren Abschnitts zugeführt wird.

6. Verfahren nach einem der Ansprüche 1 bis 5, dadurch gekennzeichnet, daß die Mischung von Olefinen eine Mischung von Butenen, Pentenen oder Hexenen umfaßt.

7. Verfahren nach einem der Ansprüche 1 bis 6, dadurch gekennzeichnet, daß die Mischung von Olefinen auch Ethylen enthält.

8. Verfahren nach einem der Ansprüche 1 bis 7, dadurch gekennzeichnet, daß der Katalysator periodisch einer Reaktivierung unterworfen wird, die aufeinanderfolgend eine Behandlung mit einem C₂-C₄-Olefin, eine Behandlung mit einem Sauerstoff enthaltenden Gas und eine Behandlung mit einem Stickstoff enthaltenden Gas umfaßt.

9. Verfahren nach Anspruch 8, dadurch gekennzeichnet, daß die Reaktivierung unter Verwendung von Ethylen durchgeführt wird.

10. Verfahren nach Anspruch 8 oder 9, dadurch gekennzeichnet, daß die Reaktivierung mit dem Olefin bei einer Temperatur von 50 bis 200°C durchgeführt wird.

11. Verfahren nach einem der Ansprüche 1 bis 10, dadurch gekennzeichnet, daß das Produkt des unteren Abschnitts der Destillation einer Co-Metathese mit einem niederen α-Olefin unterworfen wird.

12. Verfahren nach Anspruch 11, dadurch gekennzeichnet, daß als niederes α-Olefin Ethylen verwendet wird.

## Revendications

1. Procédé pour la conversion d'une oléfine ou d'un mélange d'oléfines dans une colonne de distillation dans laquelle une réaction de métathèse et une réaction d'isomérisation se produisent toutes deux, caractérisé en ce que dans la partie supérieure de la colonne une réaction de métathèse se produit et que dans la partie inférieure de la colonne une réaction combinée de métathèse et d'isomérisation se produit.

2. Procédé selon la revendication 1, caractérisé en ce que la partie inférieure de la colonne est remplie d'un catalyseur bifonctionnel qui fournit à la fois la réaction d'isomérisation et la réaction de métathèse.

3. Procédé selon la revendication 2, caractérisé en ce que, comme catalyseur bifonctionnel, on utilise de l'oxyde de rhénium ou de l'oxyde de molybdène sur un support de silice-alumine.

4. Procédé selon la revendication 1, caractérisé en ce qu'une bouillie ou une solution d'un catalyseur de métathèse est introduite dans la partie supérieure de la colonne, cette partie supérieure étant remplie d'un garnissage inerte.

5. Procédé selon l'une quelconque des revendications 1-4, caractérisé en ce qu'on introduit l'oléfine ou le mélange d'oléfines dans la colonne de distillation à un point situé au-dessus de la partie inférieure de la colonne.

6. Procédé selon l'une quelconque des revendications 1-5, caractérisé en ce que le mélange d'oléfines comprend un mélange de butènes, de pentènes ou d'hexènes.

7. Procédé selon l'une quelconque des revendications 1-6, caractérisé en ce que le mélange d'oléfines contient aussi de l'éthylène.

8. Procédé selon l'une quelconque des revendications 1-7, caractérisé en ce que le catalyseur est soumis périodiquement à une réactivation, qui comprend, successivement, un traitement avec une oléfine en C₂-C₄, un traitement avec un gaz contenant de l'oxygène et un traitement avec un gaz contenant de l'azote.

9. Procédé selon la revendication 8, catactérisé en ce qu'on effectue la réactivation en utilisant de l'éthylène.

10. Procédé selon l'une quelconque des revendications 8-9, caractérisé en ce que la réactivation avec l'oléfine est effectuée à une température de 50-200°C.

11. Procédé selon l'une quelconque des revendications 1-10, caractérisé en ce que le produit de la partie inférieure de la colonne de distillation est soumis à une co-métathèse avec une alpha-oléfine inférieure

12. Procédé selon la revendication 11, caractérisé en ce que, comme alpha-oléfine inférieure, on utilise l'éthylène.
